# EUROPEAN PATENT APPLICATION

(11) **EP 4 378 438 A1**
(43) Date of publication of application: **05.06.2024**
(21) Application number: 23213168.0
(22) Date of filing: 29.11.2023
(51) Int. Cl.: A61F 13/15, A61F 13/49, A61F 13/514, A61F 13/532, A61F 13/495, A61F 13/472, A61F 13/537

(54) **WEARABLE PART FOR WEARABLE FLUSHING DEVICE AND DIAPER PAD FOR WEARABLE PART**

(30) Priority: 30.11.2022 TW 111145932
(71) Applicant: Chin Shen Health Management Consultant Co., 114714 Taipei City (TW)
(72) Inventor: WANG, Chia-Koun, Taipei City 114714 (TW); CHANG, Shuo-Han, Taipei City 114714 (TW)
(74) Representative: HGF

(57) **Abstract**

The present disclosure provides wearable underpants used for a wearable flushing device. The wearable underpants include: a diaper pad; a waist belt combined with a buttock pad area of the diaper pad; and an abdomen sheet combined with a crotch pad area of the diaper pad. The diaper pad covers the buttocks and the crotch of a human body, the abdomen sheet covers the abdomen of the body, and the waist belt is fixed to the abdomen sheet in a covering manner so that the diaper pad closely fits and covers the buttocks and the crotch of the body when the wearable underpants are worn. The diaper pad has a recessed area corresponding to the orifices of the body, and the recessed area has a discharge opening, wherein the surface of the recessed area has a waterproof surface layer. The present application also discloses a wearable part including the above-mentioned wearable underpants and a sliding receiver, wherein the back surface of the diaper pad of the wearable underpants opposite to the surface covering the body is combined with the sliding receiver, and the sliding receiver has a first opening in a shape matching that of the discharge opening of the diaper pad.

## Description

### 1. Field of the Invention

The present invention relates to a wearable flushing device, and in particular to a wearable part for a wearable flushing device and a diaper pad for the wearable part.

### 2. Description of the Related Art

For a patient who is bedridden for an extended period of time, it is necessary for a caregiver to assist the patient in urination and defecation. Conventional nursing methods include manual placement of a waterproof pad as well as assistance and collection with a bedpan. These methods often require arduous labor for nurses, and the probability is high that the patient suffers from bedsores and even ulcers. Alternatively, another nursing method using disposable adult diapers is adopted for adult patients. Such diapers are similar to ordinary baby diapers in shape and function, but have improved breathability and comfort. However, adult diapers need to be changed manually each time. As a result, costs are high, and in treating patients who are bedridden for a long time and unable to take care of themselves, caregivers may be overworked, and sanitation cannot be ensured.

In recent years, there are automatic flushing device(s) on the market that can be worn by a bedridden patient and can automatically suck excrement (for example, urine or feces) and rinse orifices of the patient's body. However, automatic flushing device(s) or machine(s) currently available on the current market fail to provide a comfortable wearing experience for the patient. For example, a wearable part worn on the body of the patient is provided with a connector with a rigid structure disposed where the excrement of the patient is to be collected, and the connector is for connection to a suction/drying pipeline joint assembly of the automatic flushing device(s). A connector with such a rigid structure limits the caregiver's ability to place the wearable part on the patient's body. Consequently, the wearable part is not well suited to the patient's body as it limits movement. In other words, in order for the wearable part to be worn by the patient, greater effort is needed for the caregiver to move the body or the patient's limbs. In addition, due to connector being of rigid structure, it cannot be ensured that the wearable part fits securely on the body of the patient each time, and thus the risk of leakage exists. Moreover, the connector of the rigid structure of the wearable part is made of a rigid material and thus is not elastic, thereby causing discomfort and being unsuitable for use when the patient is bedridden for an extended period or is being assisted by the caregiver in other ways (for example, performing rehabilitation or moving around in a wheelchair).

Furthermore, various automatic flushing devices or machines on the market nurse the patient after excretion by using functions such as suction, flushing, and drying. However, in the current wearable part worn by patients, a completely waterproof surface is used for an excretion area (for example, the buttock area to the crotch area) of the wearable part in order to avoid as much leakage as possible during flushing, or avoid moisture retention after using the wearable part at the area in contact with the patient's skin. However, such a surface completely made of a waterproof material is unbreathable and uncomfortable. As a result, the patient often experiences humidity and stuffiness on the skin, and even suffers from rashes when wearing the wearable part. If underpants without a waterproof surface are worn, the wearable part will likely become wet when flushing occurs. In other words, the wearable part gets wet after absorbing the water when the automatic flushing device or machine rinses the body of the patient. Even though a drying procedure may be subsequently performed on the wearable part, preventing seepage into the wearable part cannot be completely avoided. As a result, moisture in wearable part causes the patient to experience discomfort.

Therefore, there is an urgent need to provide a wearable part and a wearable flushing device that are simple in structure, convenient and efficiently operated, while being sanitary and providing comfort when worn, to overcome the shortcomings of the above related art.

### SUMMARY OF THE INVENTION

An objective of the present application is to provide a wearable flushing device characterized by a simple structure, convenience, labor-saving features, sanitary operation, and intelligent functionality.

Another objective of the present application is to provide a wearable part capable of preventing water from remaining therein and keeping dryness after a wearable flushing device rinses and dries a patient, so as to provide a comfortable wearing experience.

To achieve the foregoing objectives, the present application provides a diaper pad, structured to cover the buttocks and the crotch of a human body. The diaper pad includes a recessed area. The recessed area corresponds to the orifices of the human body. The recessed area has a discharge opening. The surface of the recessed area has a waterproof surface layer.

To achieve the foregoing objectives, the present application further provides wearable underpants for a wearable flushing device. The wearable underpants include: the above-mentioned diaper pad; a waist belt spliced to an end area of the diaper pad; and an abdomen sheet spliced to the other end area of the diaper pad opposite to the end area. The diaper pad covers the buttocks and the crotch of a human body, the abdomen sheet covers the abdomen of the body, and the waist belt is fixed to the abdomen sheet in a covering manner, so that the diaper pad closely fits and covers the buttocks and the crotch of the body when the wearable underpants are worn.

To achieve the foregoing objectives, the present application further provides a wearable part for a wearable flushing device. The wearable part includes the above-mentioned wearable underpants and a sliding receiver. The back surface of the diaper pad of the wearable underpants opposite to the surface covering a human body is combined with the sliding receiver. The sliding receiver has a first opening in a shape matching that of the discharge opening of the diaper pad.

To achieve the foregoing objectives, the present application further provides a wearable flushing device, including the above-mentioned wearable part; a receptacle part detachably combined with the wearable part; and a control assembly connected to the receptacle part for automatic flushing. The sliding receiver of the wearable part is provided with a receiving slot, so that the receptacle part is detachably engaged with the receiving slot of the sliding receiver of the wearable part.

To achieve the foregoing objectives, the present application provides a method for manufacturing a diaper pad. The method includes: step 1: providing a first foam body, and attaching a first surface fabric to a first surface of the first foam body; step 2: providing a second foam body, and attaching a second surface fabric to a second surface of the second foam body; step 3: cutting the first foam body attached with the first surface fabric and the second foam body attached with the second surface fabric to respectively form a first shaped foam body and a second shaped foam body, the first shaped foam body and the second shaped foam body being formed as a shape of a diaper pad; step 4: coating a material of a waterproof surface layer on a predetermined area of the first surface fabric of the first shaped foam body; step 5: applying a joint adhesive to a third surface of the first shaped foam body opposite to the first surface with the first surface fabric, and/or applying the joint adhesive to a fourth surface of the second shaped foam body opposite to the second surface with the second surface fabric; step 6: attaching the third surface of the first shaped foam body to the fourth surface of the second shaped foam body, and performing, in a die, hot-pressing forming on the first shaped foam body and the second shaped foam body attached to each other, to form a body of the diaper pad, wherein the predetermined area of the first surface fabric coated with the waterproof surface layer forms a recessed area coated with the waterproof surface layer, and the recessed area corresponding to the orifices of a human body; and step 7: removing an excess material on the outer periphery of the body of the diaper pad in a cutting manner, and removing an excess material in a shape of a predetermined discharge opening of the recessed area on the body of the diaper pad in a penetration manner, to obtain the diaper pad.

The wearable part of the wearable flushing device in the present invention is provided with a clamping/embedding member, so that the receptacle part is detachably engaged with the wearable part. When the wearable flushing device is to be operated, a caregiver first places the wearable part on a patient, and then inserts or embeds the receptacle part into the wearable part to combine the receptacle part with the wearable part. Then, the orifices of the patient, the diaper pad of the wearable part, and the receptacle part can be automatically flushed and dried through the control assembly. In this way, manual cleaning of a patient's body by the caregiver is unnecessary, greater cleanliness and hygiene are ensured, and comfort of the patient is further improved with a special design of the diaper pad and the intelligent control of the wearable flushing device in automatic flushing. After use, it is only necessary to remove the receptacle part, and the wearable part does not need to be repeatedly put on or taken off. In addition, with the use of a structural design of the partial waterproof surface layer of the diaper pad, the patient is prevented from experiencing discomfort when wearing the wearable part for a long time.

In addition, the structure of the wearable part in the present application allows an additional small disposable diaper to be further combined with the wearable part, so as to allow the small disposable diaper to temporarily absorb excrement when the caregiver is away from the patient. The additional small disposable diaper combined with the wearable part also allows the patient to perform other activities with the help of the caregiver (for example, performing rehabilitation or going out for a sunbath in a wheelchair).

### BRIEF DESCRIPTION OF THE DRAWINGS

The following detailed descriptions are made with reference to the accompanying drawings so as to easily arrive at the present application and the various advantages thereof; the present application and the advantages thereof can be well understood accordingly.
FIG. 1 is a schematic diagram showing relationships between parts of a wearable flushing device according to an embodiment of the present application;
FIG. 2A is a structural front view of a wearable part according to an embodiment of the present application;
FIG. 2B is a structural rear view of a wearable part according to an embodiment of the present application;
FIG. 3 is a structural diagram of wearable underpants in direct contact with a human body according to another embodiment of the present application;
FIG. 4A is a structural front view of a diaper pad according to an embodiment of the present application;
FIG. 4B is a structural rear view of a diaper pad according to an embodiment of the present application;
FIG. 5A and FIG. 5B are schematic diagrams showing a combination state in which a wearable part is combined with an additional small disposable diaper according to the present application;
FIG. 6 and FIG. 7 show a combination manner of a wearable part and a receptacle part of a wearable flushing device according to an embodiment of the present application;
FIG. 8 is an exploded view of a wearable flushing device according to an embodiment of the present application, wherein the wearable underpants are omitted; and
FIG. 9 shows an exemplary manufacturing method of a diaper pad according to the present application.

### PREFERRED EMBODIMENT OF THE PRESENT INVENTION

The technical content, structural features, and effects of the present application are described in detail below with reference to the accompanying drawings.

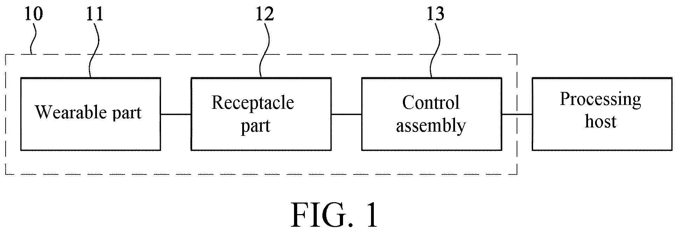

A wearable flushing device in the present application is particularly suitable for an adult patient to wear. FIG. 1 is a schematic diagram of a relationship between parts of a wearable flushing device 10 according to an embodiment of the present application. The wearable flushing device 10 includes a wearable part 11, a receptacle part 12 detachably engaged with the wearable part 11, and a control assembly 13 connected to both the receptacle part 12 and a processing host. During operation, the wearable part 11 is first worn on a patient, and then engaged with the receptacle part 12. The patient and the receptacle part 12 are automatically rinsed by the control assembly 13 without any manual operation. After flushing, it is only necessary to remove the receptacle part 12, and the wearable part 11 does not need to be repeatedly put on or taken off such that more convenience is brought to operations and nursing of a caregiver.

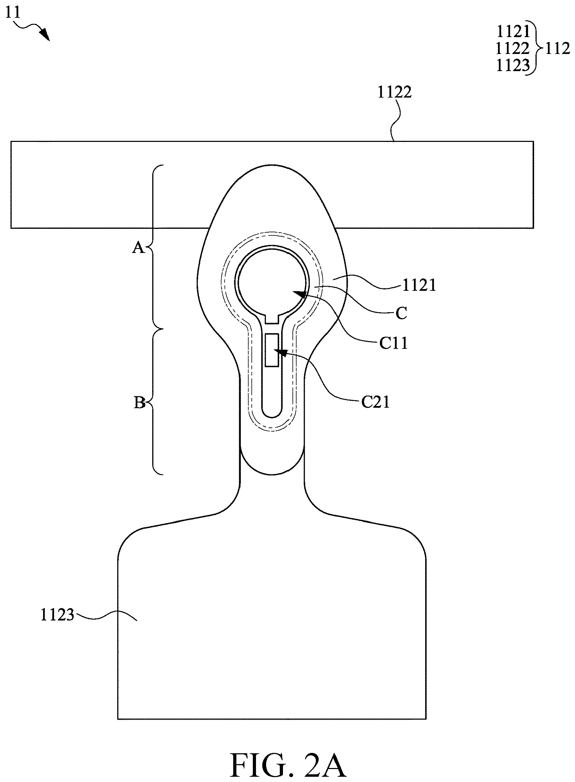

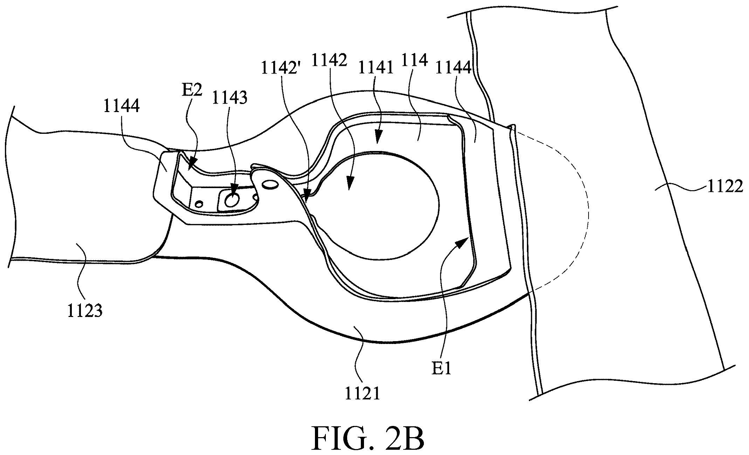

FIG. 2A is a structural front view of the wearable part 11. FIG. 2B is a structural rear view of the wearable part 11. With reference to FIG. 2A and FIG. 2B, it can be learned that the wearable part 11 includes wearable underpants 112 and a sliding receiver 114. Specifically, the wearable part 11 can be worn to cover a human body like a diaper.

The wearable underpants 112 include a diaper pad 1121, a waist belt 1122, and an abdomen sheet 1123. Specifically, the diaper pad 1121 includes a buttock pad area A and a crotch pad area B extending from and integrated with the buttock pad area A. As shown in FIG. 2A, the front side of the diaper pad 1121 is configured to directly contact the skin, and therefore a material of which the front side is made needs to be soft, elastic, comfortable, breathable, antibacterial, and the like, and should be easily bent to closely fit the skin. Therefore, the diaper pad 1121 is preferably made of a soft and elastic material including, e.g., a fabric, sponge, and a porous plastic breathable material. In this way, the buttock pad area A and the crotch pad area B of the diaper pad 1121 can be bent to closely cover the buttocks and the crotch of the human body.

In addition, as shown in FIG. 2B, the waist belt 1122 is spliced to an end area of a back surface of the diaper pad 1121 corresponding to buttock pad area A and away from the crotch pad area B, and the abdomen sheet 1123 is spliced to the other end area of the back surface of the diaper pad 1121 corresponding to the crotch pad area B and away from the buttock pad area A. The waist belt 1122, the diaper pad 1121, and the abdomen sheet 1123 together form the wearable underpants 112 that can be worn by a human body. The sliding receiver 114 is combined with the back surface of the diaper pad 1121. In this way, the wearable underpants 112 and the sliding receiver 114 form the wearable part 11 that can be worn by a patient and used for the wearable flushing device 10. When the wearable part 11 is worn by the patient, the diaper pad 1121 covers the buttocks and the crotch, the abdomen sheet 1123 covers the abdomen, and the waist belt 1122 is fixed to the abdomen sheet 1123 in a covering manner, so that the diaper pad 1121 closely fits and covers the buttocks and the crotch when the wearable underpants 112 are worn by the patient. The covering and splicing fixing manner for the above-mentioned sheets may usually be arrived at by a hook-and-loop fastener, or through direct contact and splicing by a special fabric with a characteristic of a hook-and-loop fastener.

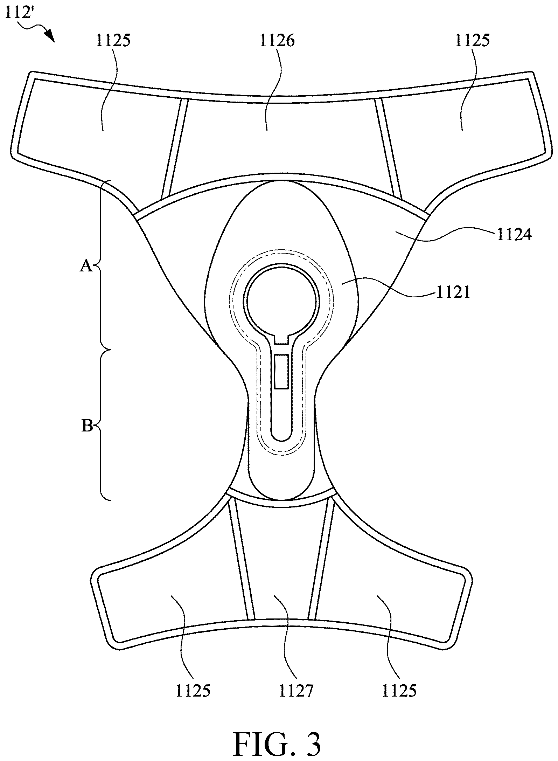

It should be understood that the wearable underpants 112 shown in FIG. 2A and FIG. 2B are merely an embodiment of the present application. The wearable underpants 112 may also have a similar design with a different covering manner. FIG. 3 shows another embodiment of the wearable underpants in direct contact with the human body. It can be learned from FIG. 3 that wearable underpants 112' also has a same diaper pad 1121 shown in FIG. 2B. The periphery of the diaper pad 1121 is connected, bonded, or pressed to the periphery of the back surface of the diaper pad 1121 through an elastic sheet 1124, so as to combine the diaper pad 1121 with the elastic sheet 1124. The upper and lower ends of the elastic sheet 1124 are further connected to an upper buttock sheet 1126 and an abdomen sheet 1127 respectively. The two side edges of the upper buttock sheet 1126 and the two side edges of the abdomen sheet 1127 are respectively connected to a plurality of elastic connecting sheets 1125, so as to form a structure of another type of wearable underpants 112' of the diaper pad 1121 shown in FIG. 3. A fabric of an entire surface layer of the wearable underpants is preferably a highly skin-friendly, antibacterial, anti-allergic, ozoneresistant and breathable material. The entire outer edge of the wearable underpants is provided with an elastic strap for adjustment for patients with different body shapes. When the wearable underpants are to be worn on the patient, the front surface of the diaper pad 1121 is also bent and adjusted, so that the buttock pad area A and the crotch pad area B of the diaper pad 1121 can be bent to cover the buttocks and the crotch of the patient. In this case, the upper buttock sheet 1126 is located at the upper buttocks of the patient. Then, the abdomen sheet 1127 is bent back to cover the patient's abdomen. Then, the connecting sheets 1125 located at the waist are stretched to be spliced, in a covering manner, to the elastic connecting sheet 1125 or the abdomen sheet 1127 that is located at the abdomen. In this way, the wearable underpants 112' are adjusted to be fixed to the body of the patient. A covering and splicing fixing manner for the above-mentioned sheets and connecting sheets may usually be implemented by a hook-and-loop fastener, or implemented through direct contact and splicing by a special fabric with a characteristic of a hook-and-loop fastener.

According to the embodiments of the wearable underpants shown in FIG. 2A and FIG. 3, it can be learned that the present application does not specifically limit the type(s) of a fixing member that fixes the diaper pad to the buttocks and the crotch of the patient. The fixing member only needs to be connected to the diaper pad 1121 and structured to fix the diaper pad 1121 to closely fit and cover the buttocks and the crotch of the human body when the diaper pad 1121 covers the buttocks and the crotch. According to the disclosed structure in the embodiment shown in FIG. 2A and FIG. 2B, the fixing member may include the waist belt 1122 combined with the buttock pad area of the diaper pad 1121 and the abdomen sheet 1123 combined with the crotch pad area of the diaper pad 1121. In the embodiment shown in FIG. 3, the fixing member may include the elastic sheet 1124 combined with the periphery of the back surface of the diaper pad 1121, the upper buttock sheet 1126 and the abdomen sheet 1127 that are combined with the upper and lower ends of the elastic sheet 1124, respectively, and the plurality of elastic connecting sheets 1125 connected to the two side edges of the upper buttock sheet 1126 and the two side edges of the abdomen sheet 1127, respectively.

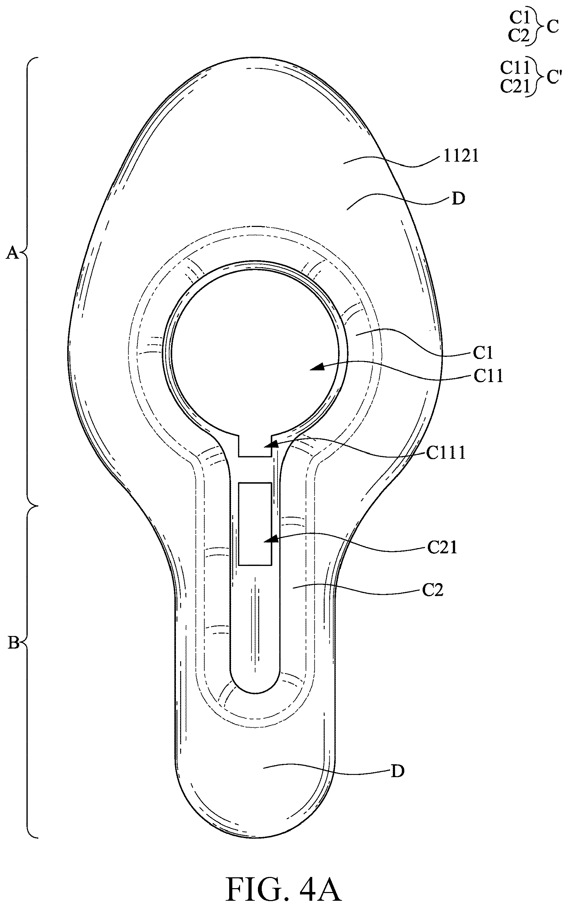

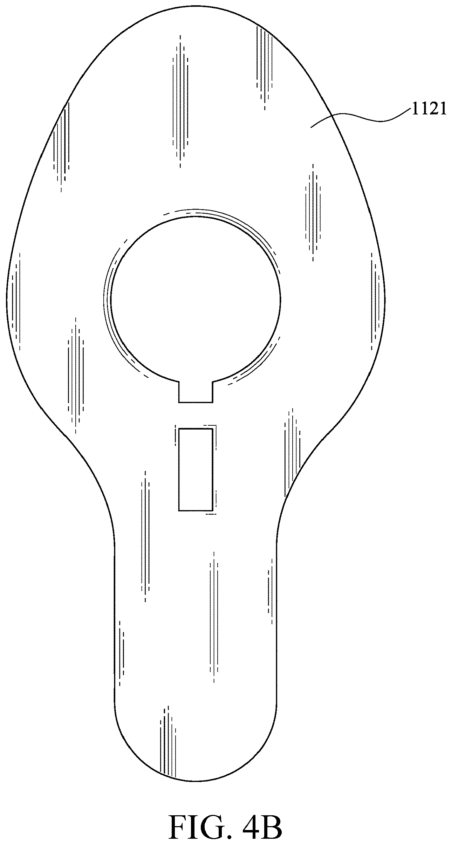

FIG. 4A is a structural front view of the diaper pad 1121 according to the present application. FIG. 4B is a structural rear view of the diaper pad 1121. As shown in FIG. 4A, the front face of the diaper pad 1121 may include a recessed area C having a waterproof surface layer thereon, and a bulge portion D surrounding the recessed area C. The recessed area C corresponds to the orifices of the human body. The recessed area C has an opening C' formed in the recessed area C. Therefore, the opening C' corresponds to the orifices of the body.

In more detail, the recessed area C of the diaper pad 1121 shown in FIG. 4A may include a first recessed area C1 substantially located at the buttock pad area A and a second recessed area C2 substantially located at the crotch pad area B. The second recessed area C2 extends from a position of the first recessed area C1 adjacent to the crotch pad area B toward a lengthwise direction of the crotch pad area B. Referring to FIG. 4B, as the back face of the diaper pad 1121 is shaped as a plane, it can be learned that the recessed area C is formed through downward sinking from the inner peripheral side of the bulge portion D of the diaper pad 1121. In other words, both the buttock pad area A and the crotch pad area B of the diaper pad 1121 have the bulge portion D at their respective portions adjacent to or surrounding the recessed area C. The bulge portion D is elastic so that the bulge portion D can be deformed to closely fit the buttocks and the crotch of the body when the buttock pad area A and the crotch pad area B of the diaper pad 1121 are adjusted and bent to cover the buttocks and crotch.

Referring to FIG. 4A again, the recessed area C has the opening C' formed in the recessed area C. The opening C' includes a discharge opening C1 1 and a flushing opening C21. The discharge opening C11 is formed in the first recessed area C1 of the recessed area C. The flushing opening C21 is formed in the second recessed area C2 of the recessed area C. In FIG. 4A, the first recessed area C1 corresponds to the position of the buttock pad area A so that the discharge opening C11 corresponds to the orifice (or defined as a first orifice) at the buttocks/anus of the human body. In addition, the second recessed area C2 corresponds to the position of the crotch pad area B, so that the position of the flushing opening C21 is designed to correspond to the orifice (or defined as a second orifice) at the crotch/urination part of the human body.

It should be noted that in the structure of the diaper pad 1121 shown in FIG. 4A, a peripheral inner wall of the recessed area C extends obliquely downwards from the bulge portion D to the bottom surface of the recessed area C. The surface of the recessed area C (that is, an area surrounded by the two dot-dashed lines in FIG. 4A), except for the opening C', has the waterproof surface layer. In other words, both the peripheral inner wall of the first recessed area C1 and the peripheral inner wall of the second recessed area C2 of the recessed area C adjacent to the bulge portion D present an inclination angle, and the surfaces of both the first recessed area C1 and the second recessed area C2, except for the discharge opening C11 and the flushing opening C21, have the waterproof surface layer.

In another embodiment, an area of the diaper pad 1121 covered by the waterproof surface layer may slightly expand outwardly from the recessed area C surrounded by the two dot-dashed lines in FIG. 4A toward the bulge portion D so as to enlarge the area of the diaper pad 1121 covered by the waterproof surface layer (for example, an area surrounded by one dot-dashed line in FIG. 4A). In other words, the surface of a partial area of the bulge portion D of the diaper pad 1121 adjacent to and surrounding the recessed area C also has the waterproof surface layer. The waterproof surface layer may be pressed on a predetermined area of the diaper pad 1121 through, for example, hot pressing. The waterproof surface layer may be made of, for example, silica gel, a thermoplastic elastomer (TPE), or thermoplastic urethane (TPU).

Referring to the back structure of the wearable part 11 shown in FIG. 2B, the back surface of the diaper pad 1121 of the wearable underpants 112 opposite to the front surface of the same for covering the human body is combined with the sliding receiver 114, so as to form the wearable part 11. In this embodiment, the back surface of the diaper pad 1121 may be in a planar shape, and is combined with the sliding receiver 114 through, for example, adhesion with an adhesive or hot-pressing. It should be understood that the diaper pad 1121 can be bent and adjusted to cover the buttocks and the crotch of the body, and therefore the sliding receiver 114 is also made of a flexible material. In this way, the sliding receiver 114 can be deformed along with the diaper pad 1121 when the latter is bent and adjusted for adaptation to the shape of the body. The sliding receiver 114 may be made of a flexible and deformable material of, for example, silica gel or a thermoplastic material, or may be obtained through three-dimensional (3D) printing by using deformable material.

In FIG. 2B, a receiving slot 1141 extending from the wide front end portion of the sliding receiver 114 to the other narrow end portion thereof is formed on the bottom side of the sliding receiver 114. A first opening 1142 and a second opening 1143 are formed in the receiving slot 1141. The shape of the first opening 1142 matches that of the discharge opening C11 of the diaper pad 1121 of the wearable underpants 112. The position of the second opening 1143 corresponds to the position of the flushing opening C21 of the diaper pad 1121. Therefore, when the sliding receiver 114 is combined with the back surface of the diaper pad 1121, the first opening 1142 of the sliding receiver 114 is aligned with and coincides with the discharge opening C11 of the diaper pad 1121, and the position of the second opening 1143 is located within the flushing opening C21 of the diaper pad 1121. In addition, the receiving slot 1141 of the sliding receiver 114 has an embedding member 1144. The embedding member 1144 is structured for the insertion of or embedding the receptacle part 12 into the receiving slot 1141 of the wearable part 11, so as to combine the receptacle part with the wearable part. In addition, the embedding member 1144 is also structured to embed and dispose an additional small disposable diaper 100 in the receiving slot 1141 so that the small diaper 100 is able to cover the first opening 1142 of the sliding receiver 114 and the discharge opening C11 of the diaper pad 1121, or preferably to simultaneously cover the first opening 1142 of the sliding receiver 114, the discharge opening C11 of the diaper pad corresponding to the first opening 1142, the second opening 1143, and the flushing opening C21 of the diaper pad corresponding to the second opening 1143.

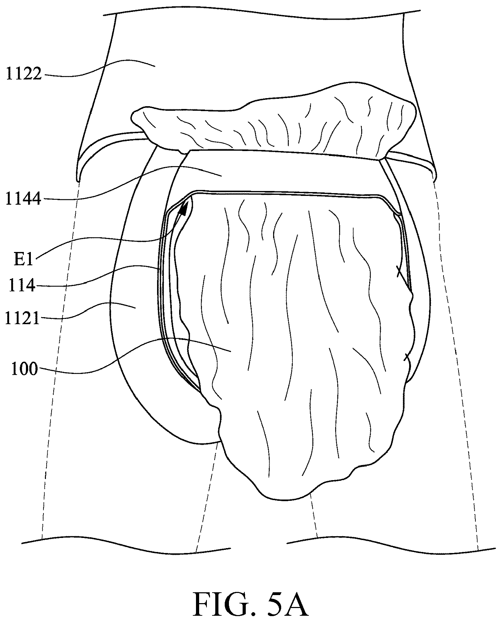

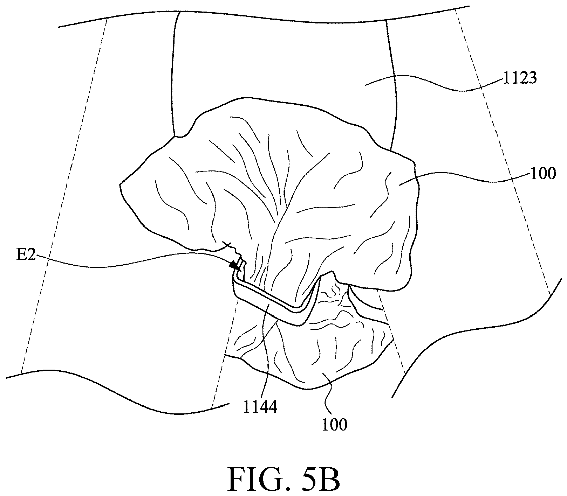

Specifically, the embedding member 1144 of the sliding receiver 114 shown in FIG. 2B includes a first embedding opening E1 formed on the front side of the receiving slot 1141 and a second embedding opening E2 formed at the tail end of the receiving slot 1141 so that the additional small diaper 100 can be disposed in the receiving slot 1141 through the two embedding openings. FIG. 5A and FIG. 5B show a state in which the wearable part 11 is worn on the human body and the additional small disposable diaper 100 is disposed in the receiving slot 1141 of the sliding receiver 114. It can be learned from FIG. 5A and FIG. 5B that the additional small disposable diaper 100 is inserted through the first embedding opening E1 and the second embedding opening E2 so that it is positioned and disposed in the receiving slot 1141, and is spread to have an unfolded portion placed in the receiving slot 1141. The unfolded portion of the additional small disposable diaper 100 covers the first opening 1142 of the sliding receiver 114, the discharge opening C11 of the diaper pad corresponding to the first opening 1142, the second opening 1143, and the flushing opening C21 of the diaper pad corresponding to the second opening 1143. In this way, excrement of the patient can be absorbed by or retained in the additional small diaper temporarily when the caregiver leaves for a moment. Also, if a patient needs to perform an activity (for example, rehabilitation or going out for an activity in a wheelchair) for a short time, the patient can be helped by the caregiver to perform the activity by using the wearable part 11 in combination with the additional small disposable diaper 100.

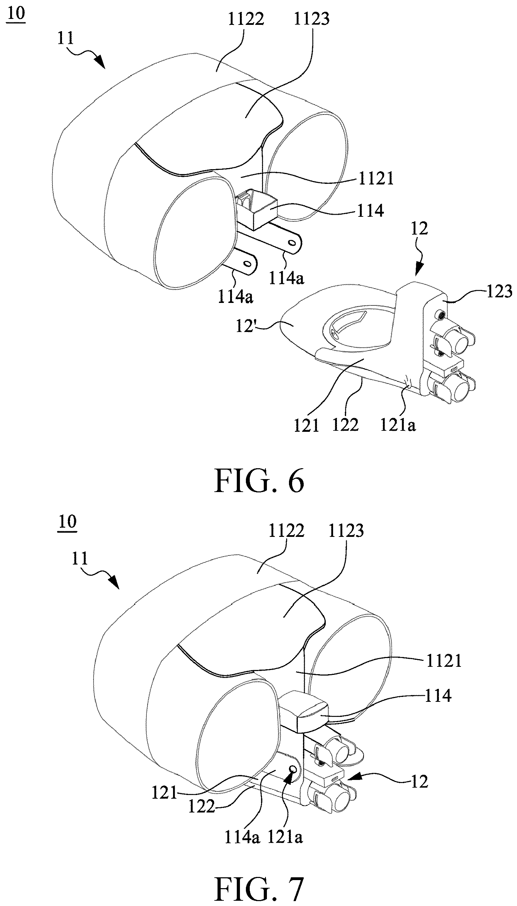

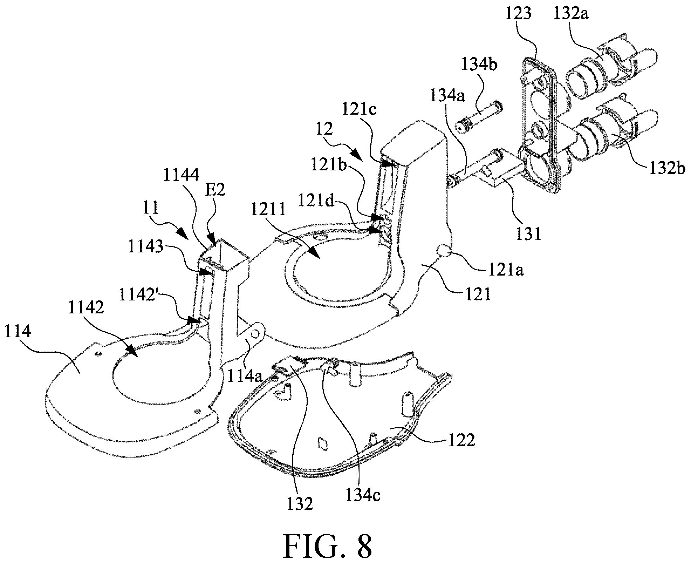

FIG. 6 and FIG. 7 show a combination manner of the wearable part 11 and the receptacle part 12 of the wearable flushing device according to the present application. FIG. 8 is an exploded view of the sliding receiver 114 and the receptacle part 12 of the wearable flushing device according to the present application. For brevity and ease of understanding, the wearable underpants having the diaper pad and combined with the sliding receiver 114 are omitted in FIG. 8. With reference to FIG. 6 to FIG. 8, it can be learned that the wearable part 11 includes the wearable underpants 112 combined with the sliding receiver 114 and being directly worn on the body, and the sliding receiver 114 is combined with the diaper pad 1121 of the wearable underpants 112. The sliding receiver 114 is configured to be connected to the receptacle part 12 of the wearable flushing device 10. For easy removal of the receptacle part 12, the receiving slot 1141 (see FIG. 2B) is formed on the bottom side of the sliding receiver 114 of the wearable part 11 so that the receptacle part 12 is detachably inserted/embedded into the receiving slot 1141 of the sliding receiver 114 of the wearable part 11. Further referring to FIG. 6 to FIG. 8, it can be clearly seen that the sliding receivers 114 are shaped and sized to match the configuration of the receptacle part 12, and thus the receptacle part 12 can be inserted/embedded into the receiving slot 1141 on the bottom side of the sliding receiver 114 from the front end 12' of the receptacle part 12 until an opening of a receptacle body 1211 of the receptacle part 12 is aligned with the first opening 1142 of the sliding receiver 114 to detachably combine the receptacle part 12 with the wearable part 11.

Referring to FIG. 8 again, the receptacle part 12 in the present application includes an upper housing 121 having the receptacle body 1211, a bottom housing 122 and a rear housing 123. Both the bottom housing 122 and the rear housing 123 are connected to the upper housing 121. The upper housing 121 and the bottom housing 122 are assembled and fastened to each other through screws. A receiving space is formed therebetween for accommodating and mounting a gravity sensor 132 and other components. The rear housing 123 is mounted behind the upper housing 121. Device(s) and pipelines are accommodated and mounted inside the rear housing for connecting with the external processing host.

Specifically, the sliding receiver 114 is provided with the embedding member 1144 for mounting and dismounting the receptacle body 1211 of the upper housing 121. In an embodiment of the present application, the front end of the structure, formed after combining the upper housing 121 having the receptacle body 1211 with the bottom housing 122, is inserted or embedded into the first embedding opening E1 of the embedding member 1144 formed on the bottom side of the sliding receiver 114 from the front end of the receptacle body 1211, and thus enters the receiving slot 1144. The upper end formed after the combination of the upper housing 121 and the rear housing 123 may be embedded into the second embedding opening E2 of the embedding member 1144 formed on the tail end of the sliding receiver 114 to fix the receptacle part within the receiving slot 1144 of the sliding receiver 114. Each of the first embedding opening E1 and the second embedding opening E2 of the sliding receiver 114 may be formed by a space between a flexible strip body, of which both ends are fixed to the peripheral edge of the sliding receiver 114 and the sliding receiver 114. The flexible strip body is stretchable so that the receptacle part 12 can be easily inserted/embedded into the sliding receiver 114 of the wearable part 11 (see FIG. 6 and FIG. 7). Such a structure has the advantage that the caregiver only needs to move the receptacle part 12, which is lightweight, to be close to the patient wearing the wearable part 11, and subsequently move the receptacle part 12 so that it is correctly combined with the sliding receiver 114 of the wearable part 11 worn on the patient. The caregiver does not need to strenuously move the body of the patient to correctly connect the wearable part worn on the patient to the receptacle part or a suction member, as is the case with the product(s) currently available on the market.

In addition, the sliding receiver 114 of the wearable part 11 may further be provided with a first connecting portion 114a, and the upper housing 121 of the receptacle part 12 is further provided with a second connecting portion 121a corresponding to the first connecting portion 114a. In an embodiment, combination of the first connecting portion 114a and the second connecting portion 121a may be carried out through, for example, a hook-and-loop fastener or a buckle member in a detachable splicing manner or detachable buckling manner. For example, the first connecting portion 114a may be a tab structure with a hole, and the second connecting portion 121a may be a protrusion structure so that the first connecting portion and the second connecting portion are detachably buckled with each other. For another example, the first connecting portion 114a and the second connecting portion 121a may be a hook-and-loop fastener structure so that the first connecting portion and the second connecting portion detachably splice with each other. With the use of the above-mentioned inserting/embedding member and the combination manner of the first connecting portion 114a of the sliding receiver 114 and the second connecting portion 121a of the upper housing 121, the sliding receiver 114 and the receptacle body 1211 can be easily and stably assembled and be easily and rapidly disassembled.

In the present application, the control assembly 13 includes a connecting element configured to be connected to the receptacle part 12 and the processing host, and detection elements for implementing some specific functions, and may further include a control member (not shown) for controlling all the elements. Preferably, the connecting element includes an air channel and a water channel. The air channel is connected to the external processing host, and is configured to supply air to the receptacle body 1211 under positive pressure or suck the excrement on the receptacle body 1211 under negative pressure. The water channel may cooperate with the air channel to rinse the patient's orifices and clean the receptacle body 1211. In this embodiment of the present application, the air channel is divided into an air supply channel 132a and a suction channel 132b (the figure shows only a part of the joints or pipelines of the air channel), and wastewater is sucked into the external processing host through the suction channel 132b. In the present application, the water channel includes a first flushing water channel 134a configured to rinse the anus (that is, the above-mentioned first orifice), a second flushing water channel 134b configured to rinse the urethra (that is, the above-mentioned second orifice of the human body), and a third flushing water channel 134c configured to clean the receptacle body 1211 (the figure only shows a part of joints or pipelines of the water channel). The first flushing water channel 134a and the second flushing water channel 134b are disposed in the receiving space between the upper housing 121 and the rear housing 125, and the outlets thereof are respectively arranged and positioned in sequence on the upper housing 121 along a vertical direction for corresponding to the positions of the orifices of the body. Specifically, as shown in FIG. 8, the outlet of the first flushing water channel 134a is disposed in a first through hole 121b formed in a recess of the upper housing 121. The second flushing water channel 134b is disposed in a second through hole 121c of another recess formed in the upper housing 121. The third flushing water channel 134c may be disposed in the receiving space between the upper housing 121 and the bottom housing 122 to optimize a spatial layout. The outlets of the respective first flushing water channel 134a, second flushing water channel 134b, and third flushing water channel 134c may protrude from the receptacle body 1121 so that flushing water jets are smoothly spurt out without hindrance by a structural body of the upper housing 121.

Preferably, in the present application, the detection element is further disposed, to improve the intelligent functionality of the product. As shown in FIG. 8, the detection elements include a camera assembly 131 mounted in an opening 121d of the upper housing 121 and protruding from the receptacle body 1211 to aim at the excrement in the receptacle body 1211 for identification. Based on the identified different types of excrement, different modes for controlling the water channel and the air channel can be adopted. In addition, the detection elements further include a gravity sensor 132 mounted in the receiving space formed between the upper housing 121 and the bottom housing 122 to detect whether the wearer's body has a rolling action or flip action. It should be noted that the mounting position of the detection elements is merely a preferred embodiment, which does not constitute a limitation.

In this embodiment of the present application, the discharge opening C11 of the diaper pad 1121 of the wearable part 11 coincides with the first opening 1142 of the sliding receiver 114, and the receptacle part 12 is combined with the wearable part 11 through the sliding receiver 114. Accordingly, the opening of the receptacle body 1211 for accommodating excrement coincides with the first opening 1142 of the sliding receiver 114. Therefore, water jet may be spurt out from the outlet of the first flushing water channel 134a arranged in the first through hole 121b of the upper housing 121 of the receptacle part 12 through the first opening 1142 of the sliding receiver 114 and the discharge opening C11 of the diaper pad 1121, thereby rinsing the first orifice of the patient. It should be noted that a small opening C111 extends from the contour of the discharge opening C11 of the diaper pad 1121 toward the crotch pad area B, and coincides with the position of a small opening 1142' extending from the contour of the first opening 1142 of the sliding receiver 114 (see FIG. 2B and FIG. 4A). Therefore, the positions of the small opening C111 of the discharge opening C11 of the diaper pad 1121 and the small opening 1142' of the first opening 1142 of the sliding receiver 114 correspond to the outlet of the first flushing water channel 134a located in the first through hole 121b of the upper housing 121 of the receptacle part 12 (see FIG. 2B, FIG. 4A, and FIG. 8). The first flushing water channel 134a may rinse the first orifice of the human body, and wastewater produced after flushing may flow into the receptacle body 1211 through the discharge opening C11 of the recessed area C of the diaper pad 1121 and the first opening 1142 of the sliding receiver 114.

In addition, the flushing opening C21 of the diaper pad 1121 of the wearable part 11 is located in an area of the second opening 1143 of the sliding receiver 114, and the receptacle part 12 is combined with the wearable part 11 through the sliding receiver 114 so that the water jet may be spurt out from the outlet of the second flushing water channel 134b arranged in the second through hole 121c of the upper housing 121 of the receptacle part 12 through the second opening 1143 of the sliding receiver 114 and the flushing opening C21 of the diaper pad 1121, thereby rinsing the second orifice of the patient. It should be noted that the flushing opening 1143 of the sliding receiver 114 may be located at a groove of the sliding receiver 114, and the contour of the groove corresponds to both the contour of the flushing opening C21 of the diaper pad 1121 and the contour of the through hole 121C in the recess of the upper housing 121 of the receptacle part 12 (see FIG. 2B, FIG. 4A, and FIG. 8). Therefore, the second flushing water channel 134b may rinse the second orifice of the human body, and wastewater produced after flushing may flow into the receptacle body 1211 through the discharge opening C11 of the recessed area C of the diaper pad 1121 and the first opening 1142 of the sliding receiver 114.

Moreover, the peripheral inner wall of the recessed area C (including the first recessed area C1 and the second recessed area C2) of the diaper pad 1121 may extend obliquely downwards from the bulge portion D to the bottom surface of the recessed area C. Therefore, the flushing wastewater produced after rinsing the first orifice and/or the second orifice of the human body can smoothly flow through the discharge opening C11 of the diaper pad 1121 along the oblique peripheral inner wall of the recessed area C, and subsequently flow into the receptacle body 1211 through the discharge opening C11 of the diaper pad 1121 and the first opening 1142 of the sliding receiver 114. The unfavorable condition wherein the wastewater remains in the diaper pad 1121 after flushing can be avoided accordingly.

In addition, in the present application, when the wearable part 11 is worn on the patient's body, because of the bulge portion D of the diaper pad 1121, the buttock pad area A and the crotch pad area B of the diaper pad 1121 can completely cover and fit the buttocks and the crotch of the patient with the soft and elastic material of the diaper pad 1121. Therefore, the boundary of the waterproof surface layer of the recessed area C of the diaper pad 1121 (i.e., the area surrounded by the two dot-dashed lines or the area surrounded by the one dot-dashed line in FIG. 4A) also fits the skin of the patient. With such configuration, the excrement of the patient or the wastewater produced, after water is spurted out from the first flushing water channel 134a to rinse the first orifice of the patient and/or spurted out from the second flushing water channel 134b to rinse the second orifice of the patient, can be resisted by the waterproof surface layer of the diaper pad 1121 to prevent the excrement/wastewater from seeping into the diaper pad 1121. In addition, with the configuration of the first recessed area C1 of the recessed area C, after excretion by the patient, the wearable part 11 of the wearable flushing device 10 is cleaned and dried so that the surface of the diaper pad 1121 in contact with the skin of the patient remains dry without the presence of water or moisture. As the body of the diaper pad 1121 is made of a soft, elastic and breathable material and only the recessed area C has the waterproof layer, the patient will not experience humidity and stuffiness on the buttocks and crotch areas covered by the diaper pad when the wearable part 11 is worn on his/her body. The patient is able to wear the wearable part for a long time without discomfort.

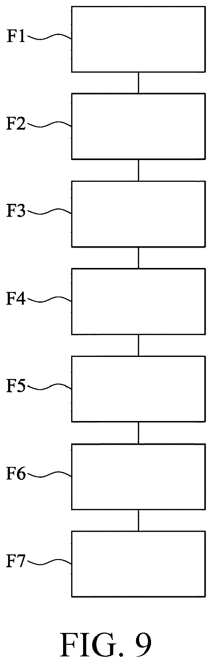

FIG. 9 shows an exemplary manufacturing method of a diaper pad according to the present application. As shown in FIG. 9, a diaper pad may be manufactured in the present application by the following steps.

Step F 1: A first foam body is provided, and a first surface fabric is attached to a first surface of the first foam body. The first foam body may be made of, for example, a high-density foam body. The first surface fabric may be, for example, an elastic fiber fabric.

Step F2: A second foam body is provided, and a second surface fabric is attached to a second surface of the second foam body. The second foam body may be made of, for example, a high-density foam body. The second surface fabric may be, for example, an elastic fiber fabric.

Step F3: The first foam body attached with the first surface fabric and the second foam body attached with the second surface fabric are cut into a first shaped foam body and a second shaped foam body, respectively, that may be formed as a shape of the diaper pad.

Step F4: Coating a material of a waterproof surface layer on a predetermined area of the first surface fabric of the first shaped foam body.

Step F5: Applying a joint adhesive to a third surface of the first shaped foam body opposite to the first surface with the first surface fabric, and/or a fourth surface of the second shaped foam body opposite to the second surface with the second surface fabric.

Step F6: The third surface of the first shaped foam body and the fourth surface of the second shaped foam body are aligned and attached to each other, and hot-pressing is performed, in a die, to the attached first shaped foam body and second shaped foam body so as to form a body of the diaper pad. A predetermined area of the first surface fabric coated with the waterproof surface layer forms a recessed area having the waterproof surface layer and corresponding to the orifices of a human body.

Step F7: Removing an excess material on the outer periphery of the body of the diaper pad in a cutting manner, and removing a material in a shape of a predetermined discharge opening of the recessed area on the body of the diaper pad in a penetration manner so as to obtain the diaper pad.

The steps for manufacturing a diaper pad in FIG. 9 described above are merely an exemplary embodiment. The above-mentioned steps do not constitute any limitations. Instead, without effecting the formation and manufacturing of the diaper pad, the above steps may be adjusted to become different manufacturing steps.

In sum, the wearable flushing device 10 in the present application has the following superior technical effects.
1. The sliding receiver 114 of the wearable part 11 of the wearable flushing device 10 in the present application is provided with the embedding member so that the receptacle part 12 is able to be detachably combined with the wearable part 11. During operation, the caregiver places the wearable part 11 on the patient, and then combines the receptacle part 12 with the wearable part 11. The patient's body and the receptacle part 12 can be automatically flushed through the control assembly 13 without a manual operation. Therefore, greater cleanliness and hygiene are ensured, and comfort of the patient is further improved in combination with the intelligent automatic control of the automatic flushing. In addition, after cleaning and drying, only the receptacle part 12 needs to be removed while the wearable part 11 does not need to be repeatedly put on or taken off. In this way, the workload and efforts in nursing the patient are reduced, mental fatigue of the caregiver or a long-term caregiver is further alleviated, or the caregiver-to-patient nursing ratio may be enhanced.
2. In the present application, the diaper pad 1121 of the wearable part 11 has the properties of being soft, elastic, comfortable, breathable, antibacterial, and easily deformed/bent to closely fit the skin of the human body. In addition, the sliding receiver 114 of the wearable part 11 for combining with the diaper pad 1121 is also made of a flexible and deformable material. Therefore, when the wearable part 11 is to be worn on the patient, the caregiver can easily place the wearable part on the body of the patient by slightly moving the patient's body, and thus physical exertion by the caregiver is greatly reduced. In addition, with the use of elastic material of the diaper pad 1121 that makes the diaper pad closely fit the skin of the body, and the use of the waterproof surface layer on the recessed area C of the diaper pad 1121, seepage or contamination on the diaper pad 1121 can be effectively prevented. The bedridden patient thus is more willing to wear the wearable part for a long time. When excretion is needed, the caregiver can perform such nursing and cleaning work by merely moving the receptacle part 12, and may easily combine it with the wearable part 11. The caregiver does not need to move the body of the patient in order to adapt to a combination step of the receptacle part 12 with the wearable pants, thereby alleviating some of the burden of caring for a long-term bedridden patient.
3. According to the configuration of the wearable part 11 in the present application, the small disposable diaper can be embedded into the receiving slot 1141 of the sliding receiver 114 so as to cover the first opening 1142 and the second opening 1143 of the wearable part 11 as well as the corresponding discharge opening C11 and the flushing opening C21 of the diaper pad 1121. Therefore, excrement of the patient can be absorbed by or retained in the small disposable diaper temporarily when the caregiver leaves for a moment, or when a patient needs to perform an activity (for example, rehabilitation or going out for an activity in a wheelchair) for a short time. The patient can be helped by the caregiver to perform such activities by using the wearable part 11 in combination with the small disposable diaper.
4. According to the configuration of the diaper pad 1121 in the present application, the diaper pad has the recessed area C with the waterproof surface layer. The peripheral edge of the waterproof surface layer also fits the skin of the patient so that the excrement or the flushing wastewater can be resisted while preventing the excrement/wastewater from seeping into another part of the diaper pad 1121. In addition, with the configuration of the first recessed area C1 of the recessed area C, when the wearable part 11 of the wearable flushing device 10 worn on the patient's body is cleaned and dried, no water or moisture remains in the diaper pad 1121 in contact with the skin of the patient. As the body of the diaper pad 1121 is made of a soft, elastic and breathable material, and only the recessed area C has the waterproof layer, the patient does not experience humidity and stuffiness on his/her buttocks and crotch areas covered by the diaper pad, and thus is willing to wear or use the wearable part for a long time.

The above descriptions merely disclose the preferred embodiments of the present application, and are certainly not intended to limit the scope of the present application. Therefore, equivalent variations made to the present application still fall within the scope of the same.

## Claims

1. A diaper pad, configured to cover the buttocks and the crotch of a human body, wherein the diaper pad comprises a recessed area, the recessed area corresponding to the orifices of the human body, the recessed area having a discharge opening, and wherein the surface of the recessed area has a waterproof surface layer.

2. The diaper pad according to claim 1, wherein the diaper pad comprises a buttock pad area and a crotch pad area, the buttock pad area and the crotch pad area of the diaper pad having a bulge portion adjacent to the recessed area, and the bulge portion being elastic and surrounding the recessed area; and when the buttock pad area and the crotch pad area of the diaper pad cover the buttocks and the crotch of the body, the diaper pad may be adjusted so that the bulge portion closely fits the buttocks and the crotch of the body.

3. The diaper pad according to claim 1 or 2, wherein the recessed area comprises a first recessed area located at the buttock pad area and a second recessed area located at the crotch pad area, the first recessed area corresponding to a first orifice of the orifices of the body, and the second recessed area corresponding to a second orifice of the orifices of the body, and wherein the second recessed area extends from a position of the first recessed area adjacent to the crotch pad area to the crotch pad area in a lengthwise direction thereof.

4. The diaper pad according to any one of claims 1 to 3, wherein the discharge opening is located at the first recessed area, and corresponds to the first orifice, and a flushing opening is located at the second recessed area, and corresponds to the second orifice.

5. Wearable underpants for a wearable flushing device, the wearable underpants comprising:
the diaper pad according to any one of claims 1 to 4; and
a fixing member connected to the diaper pad and configured to fix the diaper pad to closely fit and cover the buttocks and the crotch of the human body when the diaper pad covers the buttocks and the crotch of a human body.

6. The wearable underpants according to claim 5, wherein the fixing member comprises:
a waist belt, combined with an end area of the diaper pad; and
an abdomen sheet, combined with the other end area of the diaper pad opposite to the end area, wherein
when the diaper pad covers the buttocks and the crotch of the body, the abdomen sheet covers the abdomen of the body, and the waist belt is fixed to the abdomen sheet in a covering manner so that the diaper pad closely fits and covers the buttocks and the crotch of the body.

7. A wearable part for a wearable flushing device, the wearable part comprising:
the wearable underpants according to claim 5 or 6; and
a sliding receiver, wherein the back surface of the diaper pad of the wearable underpants opposite to the surface covering a human body is combined with the sliding receiver, and the sliding receiver has a first opening having a shape matching that of the discharge opening of the diaper pad, and wherein the sliding receiver has a second opening corresponding to a flushing opening of the diaper pad.

8. The wearable part according to claim 7, wherein the sliding receiver has a receiving slot and the sliding receiver has an embedding member disposed on the receiving slot, the embedding member being configured to allow the insertion of an additional small diaper into the receiving slot; and the additional small diaper covers the first opening of the sliding receiver and the discharge opening of the diaper pad.

9. The wearable part according to claim 7 or 8, wherein the embedding member comprises a first embedding opening and a second embedding opening formed in the receiving slot so that the additional small diaper inserts through the first embedding opening and the second embedding opening and is located in the receiving slot.

10. The wearable part according to any one of claims 7 to 9, wherein the sliding receiver is made of a flexible material.

11. A wearable flushing device, comprising:
the wearable part according to any one of claims 7 to 9;
a receptacle part, detachably combined with the wearable part; and
a control assembly, connected to the receptacle part for automatic flushing, wherein
the sliding receiver of the wearable part is provided with a receiving slot, so that the receptacle part is detachably combined with the receiving slot of the sliding receiver of the wearable part.

12. The wearable flushing device according to claim 11, wherein the receiving slot of the sliding receiver of the wearable part has an embedding member disposed thereon, the receptacle part comprising an upper housing having a receptacle body, a bottom housing and a rear housing connected to both the upper housing and the bottom housing; and the embedding member is configured to embed the upper housing and the bottom housing of the receptacle part into the receiving slot of the sliding receiver.

13. The wearable flushing device according to claim 12, wherein the embedding member comprises a first embedding opening and a second embedding opening; the upper housing and the bottom housing of the receptacle part are inserted into the receiving slot from the first embedding opening of the sliding receiver, and are further inserted into the second embedding opening of the sliding receiver so that the receptacle part and the wearable part are detachably combined with each other.

14. The wearable flushing device according to claim 12 or 13, wherein a receiving space is formed between the upper housing and the rear housing and between the upper housing and the bottom housing; the water channel is disposed in the receiving space and comprises a first flushing water channel, a second flushing water channel, and a third flushing water channel protruding into the receptacle body.

15. A method for manufacturing a diaper pad, the method comprising the following steps:
step 1: providing a first foam body, and attaching a first surface fabric to a first surface of the first foam body;
step 2: providing a second foam body, and attaching a second surface fabric to a second surface of the second foam body;
step 3: cutting the first foam body attached with the first surface fabric and the second foam body attached with the second surface fabric into a first shaped foam body and a second shaped foam body, respectively, that may be formed into the shape of a diaper pad;
step 4: Coating a material of a waterproof surface layer on a predetermined area of the first surface fabric of the first shaped foam body;
step 5: Applying a joint adhesive to a third surface of the first shaped foam body opposite to the first surface with the first surface fabric, and/or a fourth surface of the second shaped foam body opposite to the second surface with the second surface fabric;
step 6: attaching the third surface of the first shaped foam body to the fourth surface of the second shaped foam body, and performing, in a die, hot-pressing on the first shaped foam body and the second shaped foam body attached to each other to form a body of the diaper pad; a recessed area with the waterproof surface layer being formed in the predetermined area of the first surface fabric coated with the waterproof surface layer, and the recessed area corresponding to the orifices of a human body; and
step 7: removing an excess material on the outer periphery of the body of the diaper pad in a cutting manner, and removing a material in a shape of a predetermined discharge opening of the recessed area on the body of the diaper pad in a penetration manner so as to obtain the diaper pad.
